# EUROPEAN PATENT APPLICATION

(11) **EP 4 098 736 A1**
(43) Date of publication of application: **07.12.2022**
(21) Application number: 20917095.0
(22) Date of filing: 30.01.2020
(51) Int. Cl.: C12M 1/00

(54) **DNA EXTRACTION METHOD AND NAIL PROCESSOR**

(71) Applicant: NEC Corporation, 108-8001 Tokyo (JP)
(72) Inventor: ASOGAWA, Minoru, Tokyo 108-8001 (JP)
(74) Representative: Betten & Resch
(86) International application number: PCT/JP2020/003466
(87) International publication number: WO 2021/152780

(57) **Abstract**

To provide a technology that contribute to improvement in DNA extraction efficiency from a nail. There is provided a DNA extraction method, comprising a processing step for processing a nail as a sample for DNA extraction while maintaining a state of one block so as to increase a surface area of the nail to be exposed to a DNA extraction reagent, and an extracting step for exposing the processed nail to the DNA extraction reagent so as to extract DNA.

## Description

### FIELD

The present invention relates to a DNA extraction method and a nail processor.

### BACKGROUND

There is a technology in which a nail is dissolved with a reagent and then DNA is extracted from the nail (see, for example, Patent Literature 1). In such technology, a process is performed in order to improve dissolving efficiency, such as a process of pulverizing a nail into a powder with a beater beating the nail with an iron ball, etc., a process of cutting a nail with scissors finely (for example, into one-millimeter blocks), and the like.

### CITATION LIST

### PATENT LITERATURE

PTL 1: Tokkai JP 2009-11210A

### SUMMARY

### TECHNICAL PROBLEM

The following analysis is made from an aspect of the present invention. Herein, disclosure of the document cited in the Citation List is incorporated into the present application by reference thereto.

As described above, in a case where a nail is pulverized/cut and then DNA is extracted, there are various problems. For example, in a case where a nail is milled into a powder by the beater, it is required to wash the beater carefully so as to prevent carrying over/contamination into a next sample. Further, since nails pulverized into a powder would float on a DNA extraction reagent, an operation for precipitation and stirring is required. In addition, in a case where a nail is pulverized or cut, there is a problem that the nails pulverized or cut have a higher possibility of being lost. Particularly, DNA extraction from a nail has a higher difficulty when compared with other samples (for example, oral cells), such as a process using a stronger reagent or a higher temperature processing is required and the like, thus it is likely that the DNA extraction from a nail is performed in a case where it is impossible to obtain DNA from other samples. Therefore, it can be said that a nail as a sample is valuable, thus a serious problem may occur even by a little amount of loss. That is, a conventional method of DNA extraction from a nail involves a point which should be improved in an aspect of DNA extraction efficiency.

Accordingly, it is a purpose of the present invention to provide a DNA extraction method and a nail processor which contribute to improvement in efficiency of DNA extraction from a nail.

### SOLUTION TO PROBLEM

According to a first aspect of the present invention, there is provided a DNA extraction method, comprising:
a processing step for processing a nail as a sample for DNA extraction while maintaining a state of one block so as to increase a surface area of the nail to be exposed to DNA extraction reagent, and
an extracting step for exposing the processed nail to the DNA extraction reagent so as to extract DNA.

According to a second aspect of the present invention, there is provided a nail processor, which processes a nail as a sample for DNA extraction while maintaining a state of one block so as to increase a surface area of the nail to be exposed to a DNA extraction reagent.

### ADVANTAGEOUS EFFECTS OF INVENTION

According to each of the aspects of the present invention, there are provided a DNA extraction method and a nail processor which contribute to improvement in efficiency of DNA extraction from a nail.

### BRIEF DESCRIPTION OF THE DRAWINGS

Figure 1 is a flowchart indicating a flow of processes in a DNA extraction method of the present invention.
Figures 2A and 2B are figures (photographs) of an example of a nail processing.
Figure 3 is an illustration of an example of a jig.
Figure 4 is an illustration of an example of the jig.
Figures 5A and 5B are illustrations of an example of the jig.
Figure 6 is an illustration of an example of the jig and a binder material.

### MODES

A possible preferable example embodiment in the present invention is explained in detail while referring to drawings. Herein, symbols in the following description are expediently attached to each element as an explanatory aid for understanding, but not for limitation of the present invention to an illustrated configuration.

First, an outline of an example embodiment of the present invention is explained.

### [Example embodiment 1]

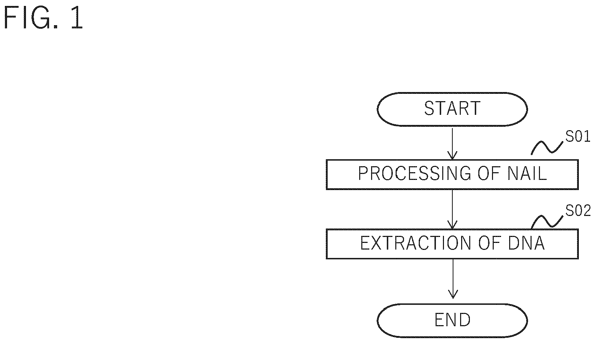

First, a basic mode of a DNA extraction method of the present invention is explained as Example embodiment 1. As shown in Figure 1, a DNA extraction method of the present invention comprises a processing step for processing a nail as a sample for DNA extraction while maintaining a state of one block (integrated piece) so as to increase a surface area of the nail to be exposed to a DNA extraction reagent (step S01). In addition, the DNA extraction method of the present invention comprises an extracting step for exposing a processed nail to the DNA extraction reagent so as to extract DNA (step S02).

A concrete example is explained. In the processing step, the nail as a sample for DNA extraction is pressed and stretched into a flat shape by a nail processor. Herein, it has been found that the nail is possible to maintain a state of one block even being pressed and stretched into the flat shape, since the nail naturally has an adequate stickiness. That is, a surface area of the nail may be increased while maintaining a state of one block when the nail is processed into a flat shape.

Next, in the extracting step, the nail processed into the flat shape is transferred into a DNA extraction container (for example, a microtube) and then exposed to a DNA extraction reagent so as to extract DNA. Herein, the nail transferred into the container is provided with a friction force [sic. surface tension] against an inner wall of the DNA extraction container, thus the nail is prevented from floating on DNA extraction reagent. In addition, even if the nail would float, the nail may be very easily immersed in the DNA extraction reagent as compared to pulverized nails, for example, the nail is pushed into DNA extraction reagent with a stick-like tool.

As described above, according to the DNA extraction method of the present invention, efficiency of DNA extraction from a nail may be improved. That is, since the nail is processed while maintaining a state of one block (integrated piece), the processed nail may be easily taken out from a processing apparatus without any loss. In other words, washing operation of the processing apparatus is performed more easily and a risk of carrying over/contamination into a next sample is reduced. In addition, since the nail still maintains a state of one block, the nail may be immersed in the DNA extraction reagent more easily.

### [Example embodiment 2]

Next, a basic mode of a nail processor of the present invention is explained as Example embodiment 2. The nail processor has a basic property of processing a nail as a sample for DNA extraction while maintaining a state of one block so as to increase a surface area of the nail to be exposed to a DNA extraction reagent. In other words, the nail processor may be adopted if it is possible to process a nail while maintaining a state of one block so as to increase a surface area.

For example, the nail processor is exemplified by a press machine that sandwiches a nail with a pair of dies and applies a pressure so that the nail is pressed and stretched into a flat shape. It is preferable that the pressure applied to the nail is of approximately 200 to 2000kgf (approximately 2000 to 20000N (newton)), but may be of 200kgf or less or 2000kgf or more. That is, if the pressure applied to the nail is increased, the surface area of the flat pressed nail will be increased whereas the thickness of the flat pressed nail will be decreased, resulting in that a demerit would occur in a point that maintenance of the one block state becomes more difficult. On the other hand, if the pressure applied to the nail will be decreased, the maintenance of the one block state becomes easier whereas a demerit would occur in a point of the increase in the nail surface area (i.e., suppressed increase). Therefore, the pressure applied to the nail may be selected depending on a variety of factors, such as operator's skill, a purpose of DNA extraction, and the like. A mechanism for applying a pressure to a nail may be selected from various mechanisms, such as a manual mechanism (for example, a mechanism using a hydraulic jack), a mechanical mechanism, and the like.

Material, configuration of the dies and the like, may be also changed as far as it is possible to process the nail while maintaining the one block state so as to increase surface area. As one example, a die made of a stainless steel and having a flat pressing surface is exemplified. Herein, the pressing surface may be uneven, that is intended to further increase the nail surface area.

Further, not only simply pressing the nail, an additional operation may be also added, such as a pair of dies is moved or rotated relative to one another, so that the nail is pressedly stretched. Here, a press surface of the die may be inclined. Here, application of the pressure to the nail, movement and/or rotation of the die(s) may be performed simultaneously or independently of one another.

Further, the mechanism (way) for applying pressure to the nail may be selected from various mechanisms. Simply applying uniform pressure to an entire nail would result in a process of nail while maintaining a state of one block so as to increase a surface area of the nail. However, an area on the nail to which the pressure is applied may be gradually changed. For example, initially the pressure is applied to a center area of the nail, and then the area of the nail to which the pressure is applied is gradually shifted to the outside. In such case, the nail is gradually stretched from the center area toward the outside, thus more efficient stretch of the nail can be achieved.

Further, the nail processor is also exemplified by a roller mechanism which sandwiches with a pair of rollers so as to roll (stretch) the nail into a flat shape. Here, spike lines along a circumference of a roller surface may be provided. For example, in a case where a sheet-shaped nail derived from a dead body is rolled (stretched) with a roller mechanism having the spike lines, stretch direction of the nail is regulated to a rotational direction of the roller (insertion direction into a gap between rollers), thus stretch of the nail in roller axis direction is restricted. Thereby, the nail is processed into a sheet configured by moderately connected-multiple strips, and thus the state of one block may be maintained more easily (for example, imaging "noshi-ika (snacks of dried squid)"). Herein, the spike lines may be oblique to a roller circumference, not only along the roller circumference.

In addition, the nail processor is also exemplified by a hammer mechanism, which stretches a nail by beating the nail with a hammer.

### [Example embodiment 3]

Next, a concrete example of a DNA extraction method and a nail processor of the present invention is explained as Example embodiment 3.

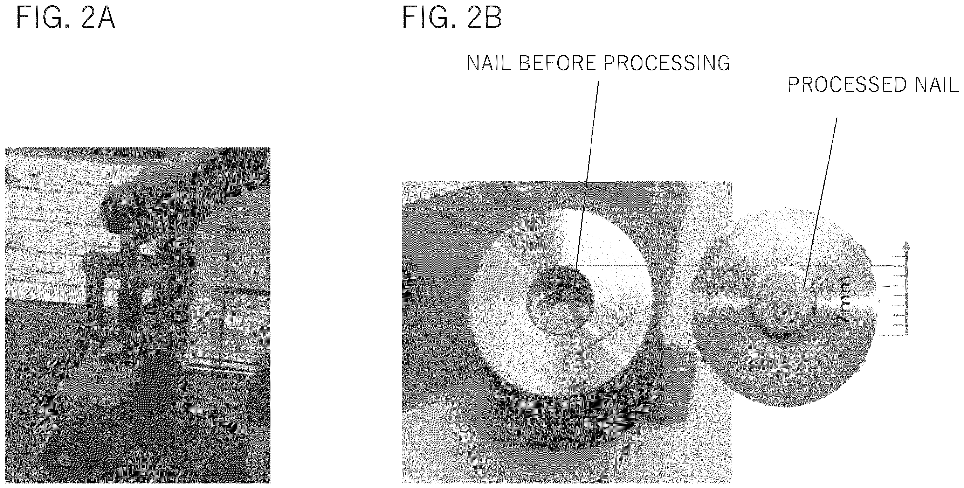

First, a nail having 1 mm of width, which has been obtained from a subject, is stretched with a press machine as a nail processor. Figure 2A shows an example of the press machine as the nail processor (a hydraulic experimental pellet press of Specac Ltd.). Dies used have a planar press surface. At that time, when pressure of about 2000 kgf is applied to the nail, the nail is processed into an ellipse sheet having about 7 mm of major axis and about 5 mm of short axis, as shown in Figure 2B.

The processed nail is detached from the die with a spatula. At that time, the nail has a hardness of an extent that makes the nail to be picked up with tweezers and has a softness of an extent that makes the nail to be bent, thus the nail can be easily detached from the die.

The nail detached from the die is inserted in a cartridge for DNA analysis (for example, RapidHIT of Thermo Fisher Scientific K.K.). The cartridge is originally so designed that a swab to which oral cells are attached may be inserted therein. Therefore, a process, such as that the nail is wound on a stick tool or the nail is cut into multiple strips, may be performed upon insertion into the cartridge. Or, the nail may be inserted forcibly.

With respect to a subsequent process of dissolving the nail and the like, since such process has been established as an ordinary experimentation method, explanation thereof is omitted. That is, in the present invention, processes until applying processed nail in place of oral cells attached to a swab are technological differences from an ordinary DNA analysis method.

### [Example embodiment 4]

Next, an application mode relating to a DNA extraction method and a nail processor of the present invention is explained as Example embodiment 4.

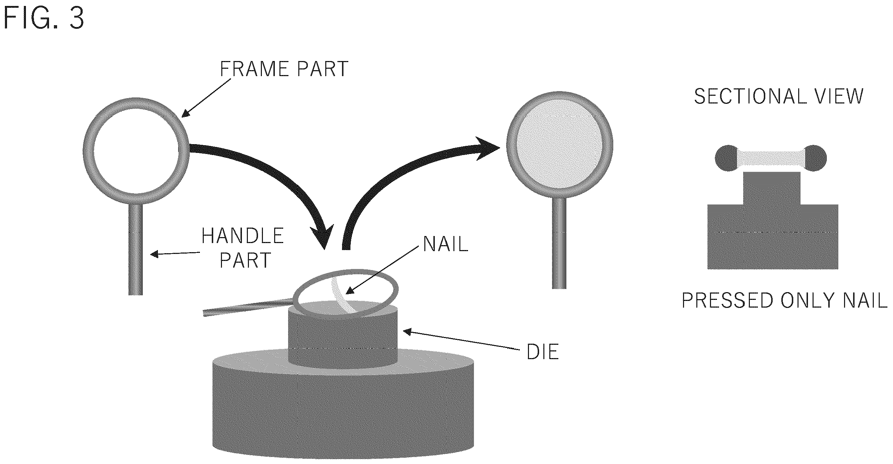

For example, in a processing step for the DNA extraction method of the present invention, a jig with a handle part may be provided so that a nail is processed integrally with the jig. Concretely, the jig with the handle part comprises a frame part having a configuration along circumference of a press surface as illustrated in, for example, Figure 3. When a nail is pressed by a press process and the nail reaches the frame part, the processed nail can be detached from the nail processor together with the jig.

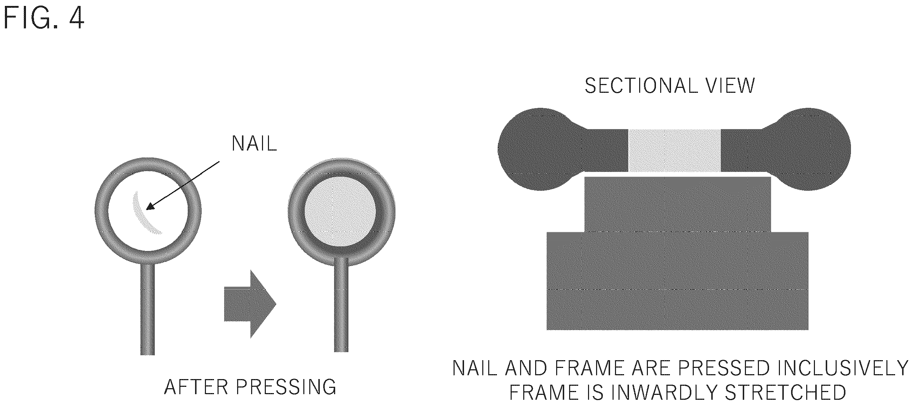

In addition, a part of the frame part may be pressed by the press process as illustrated in, for example, Figure 4. Herein, when the frame part is so designed that the pressed frame part is stretched inwardly, the nail easily contacts to the frame part.

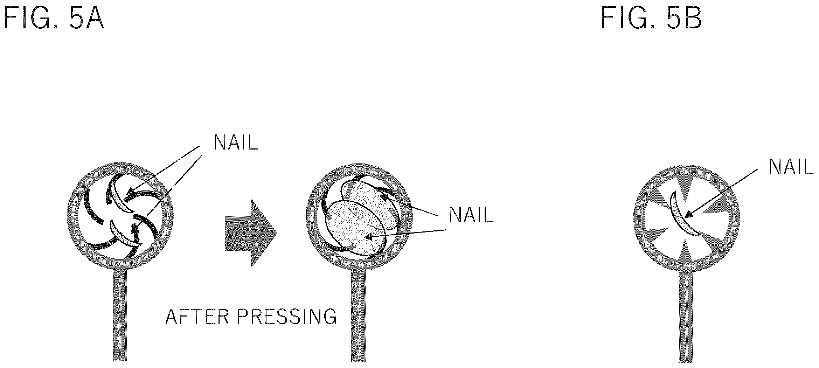

In addition, a barb (beard) part may be provided, which inwardly extends form the frame part and to be pressed together with the nail, as illustrated in, for example, Figures 5A and 5B. Such barb part may have a spiral shape as illustrated in Figure 5A, or have a radial shape as illustrated in Figure 5B.

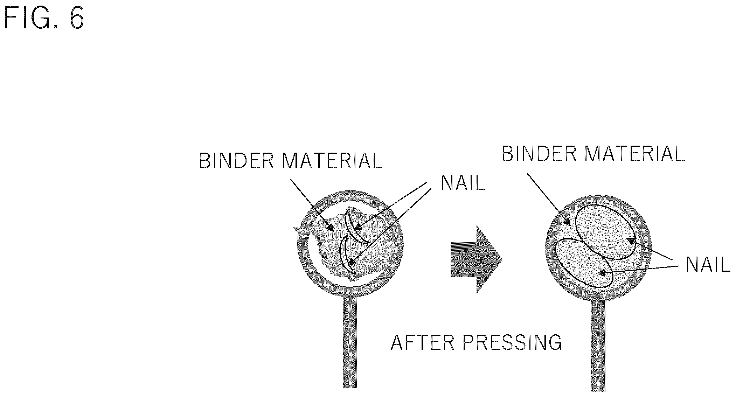

In addition, a binder material may be additionally provided as illustrated in Figure 6 so that a jig and a nail are integrally processed with the interposed binder material. The binder material preferably has a cotton shape or a mesh shape taken into account exposure of a nail to a DNA extraction reagent. In addition, the material of the binder material may comprise a natural material, such as cotton, plastic resin, plastics and the like.

In addition, in the DNA extraction method of the present invention, a sample is not limited to a nail, and a bone or a teeth may be applied thereto. Herein, since the bone and a teeth are easily crushed by a press process, it is preferable to perform the press process together with a thickener for maintaining a state of one block.

A part or entire of the above example embodiments may be described as following modes, but not limited thereto.

### (Mode 1)

A DNA extraction method, comprising:
a processing step for processing a nail as a sample for DNA extraction while maintaining a state of one block so as to increase a surface area of the nail to be exposed to a DNA extraction reagent, and
an extracting step for exposing the processed nail to the DNA extraction reagent so as to extract DNA.

### (Mode 2)

The DNA extraction method according to Mode 1, wherein the nail is processed into a flat shape in the processing step.

### (Mode 3)

The DNA extraction method according to Mode 2, wherein the nail is processed by press in the processing step.

### (Mode 4)

The DNA extraction method according to Mode 3, wherein a jig with a handle is used in the processing step so that the nail is processed into an integrated shape together with the jig.

### (Mode 5)

A nail processor, which processes a nail as a sample for DNA extraction while maintaining a state of one block so as to increase a surface area of the nail to be exposed to a DNA extraction reagent.

Herein, disclosure of the above Patent Literature is incorporated in the present application. The exemplary embodiment(s) or example(s) may be modified or adjusted within the scope of the entire disclosure of the present invention, inclusive of claims, based on the basic technical concept of the invention. In addition, a variety of combinations or selections (including partial selection or non-selection) of disclosed variety elements (each element in each claim, each element in each example embodiment or each example, etc.) may be made within the claims of the present invention. That is, the present invention, of course, may cover a variety of modifications or corrections that may be made by those skilled in the art in accordance with the entire disclosure of the present invention, inclusive of claims, and the technical concept of the present invention. Note, it is regarded as being included in matters disclosed in the present application to combine a part or entire of the cited Patent Literature(s) with the description of the present application.

## Claims

1. A DNA extraction method, comprising:
a processing step for processing a nail as a sample for DNA extraction while maintaining a state of one block so as to increase a surface area of the nail to be exposed to a DNA extraction reagent, and
an extracting step for exposing the processed nail to the DNA extraction reagent so as to extract DNA.

2. The DNA extraction method according to Claim 1, wherein the nail is processed into a flat shape in the processing step.

3. The DNA extraction method according to Claim 2, wherein the nail is processed by press in the processing step.

4. The DNA extraction method according to Claim 3, wherein a jig with a handle is used in the processing step so that the nail is processed into an integrated shape together with the jig.

5. A nail processor, which processes a nail as a sample for DNA extraction while maintaining a state of one block so as to increase a surface area of the nail to be exposed to a DNA extraction reagent.
